Europäisches Patentamt

(19) European Patent Office

Office européen des brevets

(11) Numéro de publication: **0 206 951**
**B1**

(12) # FASCICULE DE BREVET EUROPÉEN

(45) Date de publication du fascicule du brevet:
21.03.90

(21) Numéro de dépôt: 86420130.6

(22) Date de dépôt: 16.05.86

(51) Int. Cl. ⁵: **C 07 C 37/62**, C 07 C 37/11,
C 07 C 37/18, C 07 C 39/24,
C 07 C 211/45, C 07 C 209/68

(54) **Procédé de perfluoroalkylation de dérivés aromatiques.**

(30) Priorité: 22.05.85 FR 8507695
25.10.85 FR 8515857

(43) Date de publication de la demande:
30.12.86 Bulletin 86/52

(45) Mention de la délivrance du brevet:
21.03.90 Bulletin 90/12

(84) Etats contractants désignés:
AT BE CH DE FR GB IT LI LU NL SE

(56) Documents cité:
FR-A-2 301 511
FR-A-2 307 785
GB-A-840 725

(73) Titulaire: RHONE-POULENC CHIMIE
25, quai Paul Doumer
F-92408 Courbevoie Cédex (FR)

(72) Inventeur: Wakselman, Claude
5, rue Chanez
F-75016 - Paris (FR)
Inventeur: Tordeux, Marc
1, rue Seignelay
F-92330 - Sceaux (FR)

(74) Mandataire: Le Pennec, Magali
RHONE-POULENC INTERSERVICES Service Brevets
Chimie 25, Quai Paul Doumer
F-92408 Courbevoie Cédex (FR)

LIBERGRAF, STOCKHOLM 1990

2

## Description

La présente invention concerne un procédé de perfluoroalkylation de dérivés aromatiques. Elle concerne plus particulièrement un procédé de perfluoroalkylation de dérivés aromatiques par des halogénures de perfluoroalkyle.

Il est connu, d'après J. FUCHIKAMI, I. OJIMA (J. Fluorine Chemistry 1983, 22, 541) de perfluorer des amines aromatiques ou des phénols par des iodures ou des bromures de perfluoroalkyle dont la chaîne alkyle contient au moins trois atomes de carbone en présence de cuivre métallique comme catalyseur. La trifluorométhylation de la parachloroaniline à l'aide de l'iodure de trifluorométhyle ne donne que des traces de trifluorométhylparachloroaniline. L'iodure de trifluorométhyle n'étant pas industriel, le rendement de la réaction étant très faible cette méthode n'est pas transposable au stade industriel. La trifluorométhylation des phénols et de leurs dérivés n'est pas décrite.

Il est également connu d'après le brevet européen n° 114 359 de perfluoroalkyler des dérivés aromatiques et en particulier l'anisole (exemple 9) à l'aide d'iodure de perfluoroalkyle à longue chaîne, pendant 30 heures à 170°C et en présence d'un catalyseur à base de ruthénium. La trifluorométhylation n'est jamais décrite dans ce brevet comme dans l'article précédent. L'iodure de trifluorométhyle n'étant pas industriel, cette méthode de perfluoroalkylation n'est pas envisageable.

Il est encore connu d'après le brevet anglais n° 840 725 de préparer des composés aromatiques perfluoroalkyles par condensation d'un dérivé aromatique et d'un iodure de perfluoroalkyle contenant 1 à 12 atomes de carbone à une température comprise entre 200 et 350°C.

Les températures de condensation sont trop élevées et les durées de réaction très longues pour pouvoir envisager une exploitation industrielle économique rentable.

Les procédés classiques de perfluoroalkylation tels que ceux décrits dans le brevet EP-8 453 ne sont pas applicables aux phénols car on obtient alors une perfluoroalkylation du groupe hydroxyle et non du noyau aromatique.

La présente invention a su vaincre ces problèmes techniques et a pour objet un procédé de perfluoroalkylation de dérivés aromatiques caractérisé en ce que, dans une première étape, on met en présence ledit dérivé aromatique, du dioxyde de soufre, un métal choisi parmi le zinc, l'aluminium, le manganèse et le cadmium dans un solvant aprotique polaire; dans une deuxième étape, on ajoute un bromure ou un iodure de perfluoroalkyle éventuellement en mélange avec du dioxyde de soufre et on maintien à une température comprise entre -20°C et 115°C.

On entend au sens de la présente invention par dérivés aromatiques tout composé répondant à la formule générale (I):

$$Ar-(-R)n \qquad (I)$$

dans laquelle:

– Ar représente un radical aromatique mno ou polycyclique, hétérocyclique,
– R représente au moins un substituant choisi parmi l'hydrogène, le chlore, le brome, les radicaux alkyle linéaires, ramifiés, cycliques saturés ou insaturés éventuellement substitués, éther, alcoxy éventuellement substitué, aryl, éventuellement substitué, aryloxy, alkylthio, arylthio, amino, hydroxy, carboxylate, acyloxy, ester, amido, nitrile, acide,
– n est un nombre entier égal à 1,2 ou 3.

Pour une meilleure mise en oeuvre de l'invention on préfère utiliser des dérivés aromatiques de formule (I) dans lesquels Ar représente un radical aromatique monocyclique et $R_1$ représente au moins un substituant choisi parmi les radicaux amino ou hydroxy.

Parmi les dérivés aromatiques de formule (I) utilisées dans le procédé de l'invention on peut citer, mais de façon non limitative:

– le benzène, le toluène, le métaxylène, l'oxyde de phényle, le biphényle, le bromobenzène, le chlorobenzène, l'alpha chlorotoluène, l'alcool benzylique, le phényl acétate d'éthyle, la pyridine, la méthyl-2 pyridine;
– les amines telles que notamment l'aniline, les méthylanilines, les phénoxyanilines, les phénylanilines, les chloroanilines, les méthoxyanilines, l'aminonaphtalène, les diaminobenzènes, l'amino-3 pyridine;
– les dérivés phénoliques parmi lesquels on peut citer les phénols, les crésols, les phénylphénols, les chlorophénols, les aminophénols, les anisoles, les méthoxyphénols, les dihydroxybenzènes, le terbutyl-4 phénol, le terbutyl-3 phénol, l'hydroxy-2 pyridine.

Selon le procédé de l'invention, le bromure ou l'iodure de perfluoroalkyle répond de préférence à la formule générale (II) suivante

$$C_nF_{2n+1}X \qquad (II)$$

dans laquelle

– n est un nombre entier compris entre 1 et 12,
– X représente Br ou I.

D'un point de vue économique il est préférable d'utiliser, lorsque n est égal à 1, le bromure de trifluorométhyle et, lorsque n est supérieur à 1, les iodures de perfluoroalkyle.

En effet, le bromure de trifluorométhyle est un gaz extincteur (M.R.C. "GERSTENBERGER, A. HAAS Angew. Chem. Int." Ed. 1981, 20, 647) qui est un produit industriel fabriqué à large échelle donc d'un coût tout à fait accessible pour l'industrie. L'iodure de trifluorométhyle n'étant pas industriel n'est disponible qu'à un prix qui le rend tout à fait inutilisable. Par contre, dès que la chaîne alkyle a au moins 2 atomes, les iodures de perfluoroalkyle se présentent sur le marché à des prix nettement Inférieurs à ceux de leurs homologues bromés.

On préfère, parmi les métaux, choisir ceux qui ne présentent qu'un seul degré d'oxydation et tout particulièrement ceux dont le potentiel d'oxydo-réduction en milieu basique est compris entre - 0,8 et - 2 volts.

Ainsi sont utilisés le zinc, l'aluminium, le manganèse et le cadmium.

Parmi ces derniers, d'un point de vue économique, il est avantageux de choisir le zinc.

Le métal est avantageusement utilisé sous forme dispersée de façon à assurer le meilleur contact avec les gaz mis en oeuvre dans le procédé de l'invention. La forme et la dimension des particules de métal seront adaptées par l'homme de l'art à la réactivité des produits mis en oeuvre.

Le solvant choisi doit, autant que possible, permettre de solubiliser le dioxyde de soufre et l'halogénure de perfluoroalkyle. Répondent à cette condition, les solvants aprotiques polaires et parmi eux préférentiellement:

- l'acétonitrile
- le diméthylformamide (D.M.F.)
- le diméthylacétamide (D.M.A.)
- l'hexaméthylphosphoramide (H.M.P.A.)
- la N-méthylpyrrolidone (N.M.P.)
- le diméthylsulfoxyde (DMSO).

Le diméthylformamide et le diméthylsulfoxyde sont utilisés encore plus préférentiellement.

Pour une mise en oeuvre plus rapide du procédé, on préfère ajouter, lors de la première étape, une base organique et/ou minérale. Parmi les bases minérales, on peut citer notamment la soude, la potasse, la chaux, les métabisulfites alcalins. On préfère utiliser le métabisulfite de sodium.

Parmi les bases organiques, on peut citer les pyridines et plus particulièrement la pyridine et les méthylpyridines.

Selon un mode tout à fait privilégié de mise en oeuvre de l'invention, on utilise un rapport molaire du métal au dérivé aromatique compris entre 0,05 et 1 et de préférence compris entre 0,10 et 0,20; un rapport molaire de l'halogénure de perfluoroalkyle au dérivé aromatique de préférence supérieur ou égal à 1. Si l'halogénure de perfluoroalkyle est mis en excès et que l'halogénure utilisé est le bromure de trifluorométhyle, celui-ci sera aisément recyclé car il présente sous forme gazeuse.

Le rapport molaire du dioxyde de soufre au dérivé aromatique mis en oeuvre est de préférence compris entre 0,05 et 1,5.

Lorsqu'on met en oeuvre une base minérale, on préfère l'utiliser selon un rapport molaire base minérale au dérivé aromatique compris entre 0,4 et 1 et lorsqu'on met en oeuvre une pyridine, on préfère l'utiliser selon un rapport molaire pyridine au dérivé aromatique compris entre 0,5 et 1,5.

La température de mise en oeuvre de la réaction est comprise de préférence entre 0 et 90°C.

Il est avantageux d'utiliser une pression comprise entre 1 et 10 bars. La pression sera simplement adaptée par l'homme de l'art aux réactifs mis en présence.

On opère de préférence en l'absence d'oxygène.

On peut citer parmi les produits obtenus par le procédé de la présente invention:

- le trifluorométhylbenzène,
- les trifluorométhyltoluènes, les diméthyltrifluorométhylbenzènes, les trifluorométhylphénoxybenzènes, les trifluorométhylbiphényles, les bromotrifluorométhylbenzènes, les chlorotrifluorométhylbenzènes, les trifluorométhyl-(chlorométhyl)benzènes, les alcools trifluorométhylbenzyliques, le (trifluorométhylphényl)-acétate d'éthyle, les trifluorométhylpyridines, les méthyltrifluorométhylpyridines, les trifluorométhylanilines, les méthyl, méthoxy, chloro, phényl, phénoxytrifluorométhylanilines, les diaminotrifluorométhylbenzènes, les diaminoditrifluorométhylbenzènes, les trifluorométhyl-N,N diéthylanilines, les pentafluoroéthylanilines, les perfluorobutylanilines, les aminotrifluorométhylpyridines, les trifluorométhylphénols, les chlorotrifluorométhylphénols, les méthyltrifluorométhylphénols, les méthoxytrifluorométhylphénols, les aminotrifluorométhylphénols, les terbutyltrifluorométhylphénols, les hydroxytrifluorométhylpyridines.

Les produits objets du procédé de l'invention sont notamment utilisés comme intermédiaires de synthèse dans l'industrie pharmaceutique ou phytosanitaire.

L'invention va être plus complètement décrite à l'aide des exemples suivants qui ne devront pas être considérés comme limitatifs de l'invention.

### Exemple 1

Dans un appareil en verre épais on introduit:

- 40 g de benzène
- 100 ml de diméthylformamide
- 40 ml de méthyl-2 pyridine,
- 5 g de zinc et 20 g de métabisulfite de sodium.

On fait le vide dans l'appareil puis on le thermostate à 65°C. On introduit 20 g de dioxyde de soufre. Le mélange est agité pendant 3 heures sous une pression de bromotrifluorométhane qui est maintenue entre 8 et 7 atmosphères. L'appareil après filtration est ensuite ouvert, le mélange réactionnel est versé dans 200 g de glace et 60 ml d'acide chlorydrique concentré. Le mélange est extrait à l'éther. On obtient après évacuation des solvants et distillation 12,7 g de trifluorométhylbenzène (rendement 17 %) – delta$_F$ = - 64 ppm Eb 102°C.

## Exemple 2

On procède comme à l'exemple 1 en introduisant:

- 40 g de toluène
- 100 ml de diméthylformamide
- 40 ml de méthyl-2 pyridine
- 5 g de zinc
- 20 g de métabisulfite de sodium

On introduit 20 g de dioxyde de soufre.

Après 3 heures de réaction et extraction à l'éther on obtient par distillation et identification par chromatographie en phase gazeuse:

- 15,3 g de trifluorométhyltoluènes (Eb 145°C) soit
  • 9 % de trifluorométhyl-2 toluène
  $delta_F$ - 60,6 ppm $delta_H$ 2,47 ppm (3 H, q, J : 1 Hz) 7,24 ppm (2 H, m) 7,4 ppm (1 H, t, J : 7,5 Hz) 7,6 ppm (d, J : 7,5 Hz)
  • 4 % de trifluorométhyl-3 toluène
  $delta_F$ - 61,6 ppm $delta_H$ 2,41 ppm (3 H, s) 7,45 ppm (2 H, m) 7,35 ppm (2 H, m)
  • 9 % de trifluorométhyl-4 toluène
  $delta_F$ - 61,8 ppm $delta_H$ 2,4 ppm (3 H, s) 7,25 ppm (2 H, d, J: 8,1 Hz) 7,5 ppm (2 H, d) et

- 1,5 de trifluorométhyl-6 méthyl-2 pyridine (rendement: 2 %)
  $delta_F$ - 68 ppm $delta_H$ 2,64 ppm (3 H, s) 7,3 ppm (1 H, d) 7,46 ppm (1 H, d) 7,73 ppm (1 H, t, J: 7,75 Hz).

## Exemple 3

On procède comme à l'exemple 1 en remplaçant le benzène par 40 g de métaxylène.

Après 3 heures de réaction et extraction à l'éther on obtient après distillation du xylène et identification par chromatographie en phase gazeuse:

1) diméthyl 1,3 trifluorométhyl-5 benzène
   $delta_F$ - 61,3 ppm (s) $delta_H$ 2,37 ppm (6 H, s) 7,27 ppm (3 H)
2) diméthyl 2,4 trifluorométhyl-1 benzène rendement: 5 %
   $delta_F$ - 60,3 ppm (s large) $delta_H$ 2,3 ppm (3 H, s) 2,43 ppm (3 H, s large) 7 ppm (2 H, m) 7,53 ppm (1 H, m)
3) diméthyl-1,3 trifluorométhyl-2 benzène
   $delta_F$ - 53 ppm (sept., d, J: 3,5 Hz) $delta_H$ 2,5 ppm (q, J: 3,5 Hz) 7,13 ppm (3 H, m)

## Exemple 4

Dans un flacon en verre épais, on introduit 10 g d'oxyde de biphényle 25 ml de diméthylformamide, 10 ml de méthyl-2 pyridine, 1 g de zinc et 8 g de métabisulfite de sodium. On fait le vide dans la flacon. Celui-ci est alors thermostaté à 60°C. On introduit 4 g de dioxyde de soufre et le flacon est agité pendant 3 heures sous une pression de bromotrifluorométhane qui est maintenue entre 3,7 et 2,5 atmosphères. Le flacon est alors ouvert. On ajoute 50 g de glace, 15 ml d'acide chlorhydrique concentré. Le mélange est extrait à l'éther, la phase éthérée est rincée avec de l'acide chlorhydrique dilué et une solution de chlorure de sodium puis séchée avec du sulfate de magnésium. On obtient par distillation et chromatographie:

1) 8 % de trifluorométhyl-3 phénoxybenzène
   $delta_F$ - 62 ppm $delta_H$ 7,5 6,8 ppm (m)
2) 11 % de trifluorométhyl-2 phénoxybenzène
   $delta_F$ - 60,9 ppm - $delta_H$ 7,65 ppm (1 H d, J: 8,3 Hz) 6,91 ppm (1 H, d, J: 8,4 Hz) 7,44 - 7 ppm (7 H, m)
3) 11 % de trifluorométhyl-4 phénoxybenzène
   $delta_F$ - 61 ppm - $delta_H$ 7,54 ppm (2 H, d) 7,02 ppm (2 H, d, J: 8,7 Hz) 7,44 - 7 ppm (5 H, m)

## Exemple 5

On procède comme à l'exemple 1 en remplaçant le benzène par 40 g de biphényle.

Après 3 heures de réaction, extraction à l'éther et sublimation à 40°C on obtient:

- 14 g d'un mélange de 3 trifluorométhylbiphényles - rendement 18 % (F : 45°C) $delta_F$ - 56,7 ppm 17 %;
  $delta_F$ - 62,3 ppm 28 %; $delta_F$ - 62,2 ppm 55 %

## Exemple 6

On procède comme à l'exemple 1 en remplaçant le benzène par 40 g de bromobenzène.

Après 3 heures de réaction, on obtient après extraction à l'éther et identification par chromatographie en phase gazeuse:

- 3 % bromo-2 trifluorométhylbenzène - $delta_F$ - 61,8 ppm
- 1 % bromo-3 trifluorométhylbenzène - $delta_F$ - 61,7 ppm
- 3 % bromo-4 trifluorométhylbenzène - $delta_F$ - 61,8 ppm

## Exemple 7

On procède comme à l'exemple 1 en remplaçant le benzène par 40 g de chlorobenzène.

Après 3 heures de réaction, on obtient après extraction à l'éther et identification par chromatographie en phase gazeuse:

- 3 % chloro-2 trifluorométhylbenzène - $delta_F$ -

62,4 ppm
- 1 % chloro-3 trifluorométhylbenzène - deltaF -
  61,7 ppm
- 3 % chloro-4 trifluorométhylbenzène - delta -
  61,8 ppm

## Exemple 8

On procède comme à l'exemple 1 en remplaçant le benzène par 40 g de d'alpha-chlorotoluène.

Après 3 heures de réaction, on obtient après extraction à l'éther et identification par chromatographie en phase gazeuse:

- 3 % trifluorométhyl-2 chlorométhylbenzène -
  deltaF - 57 ppm
- 6 % trifluorométhyl-3 chlorométhylbenzène -
  deltaF - 61,6 ppm
- 6 % trifluorométhyl-4 chlorométhylbenzène -
  deltaF - 61,8 ppm

## Exemple 9

On procède comme à l'exemple 1 en remplaçant le benzène par 40 g d'alcool benzylique.

Après 3 heures de réaction, on obtient après extraction à l'éther et identification par chromatographie en phase gazeuse:

- 6 % trifluorométhyl-2 benzylalcool - deltaF - 60
  ppm
- 6 % trifluorométhyl-3 benzylalcool - deltaF -
  61,6 ppm
- 6 % trifluorométhyl-4 benzylalcool - deltaF -
  61,7 ppm

## Exemple 10

On procède comme à l'exemple 1 en remplaçant le benzène par 40 g de phénylacétate d'éthyle.

Après 3 heures de réaction, on obtient après extraction à l'éther et identification par chromatographie en phase gazeuse:

- 3 % (trifluorométhyl-2 phényl) acétate d'éthyle
  - deltaF - 59,3 ppm
- 6 % (trifluorométhyl-3 phényl) acétate d'éthyle
  - deltaF - 61,6 ppm
- 6 % (trifluorométhyl-4 phényl) acétate d'éthyle
  - deltaF - 61,8 ppm

## Exemple 11

On procède comme à l'exemple 1 en remplaçant le benzène par 50 g de pyridine sans méthyl-2 pyridine.

Après 3 heures de réaction, on obtient par distillation:

- 3 % trifluorométhyl-3 pyridine - deltaF - 61,7
  ppm Eb 108° - 110°C

- 1 % trifluorométhyl-4 pyridine - delta - 64,2
  ppm Eb 110° - 113°C
- 5 % trifluorométhyl-2 pyridine - deltaF - 67,7
  ppm Eb 140°C

## Exemple 12

On procède comme à l'exemple 1 en supprimant le benzène.

Après 3 heures de réaction, on obtient par extraction à l'éther:

1) méthyl-2 trifluorométhyl-4 pyridine (rendement 1 %)
   deltaF - 63,8 ppm deltaH 2,67 ppm (3 H, s)
   7,36 ppm (1 H, d) 7,45 ppm (1 H) 8,77 ppm (1
   H, d, J : 5,5 Hz)
2) méthyl-2 trifluorométhyl-3 pyridine (rendement 4 %)
   deltaF - 62,2 ppm (s large) deltaH 2,89 ppm (3
   H, s large) 7,29 ppm (1 H, d, J : 8 Hz) 7,84
   ppm (1 H, d x d) 8,62 ppm (1 H, d, J : 4,75
   Hz)
3) méthyl-2 trifluorométhyl-5 pyridine (rendement 3 %)
   deltaF -62 ppm (s) deltaH 2,62 ppm (3 H, s)
   7,86 ppm (1 H) 8,2 ppm (1 H, d, J : 8 Hz) 8,77
   ppm (1 H, d)
4) méthyl-2 trifluorométhyl-6 pyridine (rendement 2 %)
   deltaF - 68 ppm (s) deltaH 2,63 ppm (3 H, s)
   7,4 ppm (1 H, d, J : 7,7 Hz) 7,52 ppm (1 H, d,
   7,7 Hz) 7,84 ppm (1 H, t)

## Exemple 13

Dans un flacon en verre épais, on introduit 10 g (0,11 mole) d'aniline, 25 ml de diméthylformamide et 1 g de zinc.

Le flacon est placé dans un appareil de Parr.

On fait le vide dans le flacon. On introduit 8 g de dioxyde de soufre. Le flacon est agité pendant 3 heures sous une pression de bromotrifluorométhane qui est maintenue entre 3,7 et 2,4 atmosphères. La réaction est exothermique. Le flacon est ensuite ouvert. On ajoute 50 g de glace, 15 ml d'acide chlorhydrique concentré. Le mélange est extrait à l'éther. On obtient par distillation:

1) 3,4 g de trifluorométhyl-2 aniline (rendement 20 %)
   deltaF = - 63 ppm Eb14 mm Hg 66°C
2) 1,7 g de trifluorométhyl-4 aniline (rendement 10 %)
   deltaF = - 60 ppm Eb14 mm Hg 86°C

## Exemple 14

On procède comme à l'exemple 13 en introduisant:

- 10 g (0,11 mole) d'aniline

## 9

- 25 ml de diméthylformamide
- 1 g de zinc
- 10 ml de méthyl-2 pyridine
- 6 g de soude en poudre Après 3 heures de réaction et extraction à l'éther, on obtient par distillation:

- 4,8 g de trifluorométhyl-2 aniline (rendement: 28 %)
- 2,6 g de trifluorométhyl-4 aniline (rendement: 15 %)

### Exemple 15

On procède comme à l'exemple 14 en remplaçant la soude par 10 g de métabisulfite de sodium en poudre.

Après 2 heures de réaction et extraction à l'éther, on obtient:

- 6,2 g de trifluorométhyl-2 aniline (rendement 36 %)
- 3,4 g de trifluorométhyl-4 aniline (rendement 20 %)

### Exemple 16

On procède comme à l'exemple 13 en introduisant:

- 10 g (0,093 mole) de méthyl-2 aniline
- 25 ml de diméthylformamide
- 1 g de zinc
- 10 ml de méthyl-2 pyridine

Après 3 heures de réaction et extraction à l'éther, on obtient 4,5 g d'un mélange qui est séparé par chromatographie en phase gazeuse, on obtient:

- méthyl-2 trifluorométhyl-6 aniline (rendement 15 %)
  delta$_F$: - 62 ppm delta$_H$: 7,33 ppm (t, 2H) 6,73 ppm (t, 1H) J = 8,5 Hz
- méthyl-2 trifluorométhyl-4 aniline (rendement 15 %)
  delta$_F$: - 60 ppm delta$_H$: 7,33 ppm (m, 2H) 6,73 ppm (d, 1H) J = 8,5 Hz

### Exemple 17

On procède comme à l'exemple 16 en remplaçant la méthyl-2 aniline par 10 g de méthyl-4 aniline et la méthyl-2 pyridine par 10 ml de diméthyl-2,6 pyridine.

Après 3 heures de réaction, on obtient 4,9 g (0,028 mole) de méthyl-4 trifluorométhyl-2 aniline (rendement 30 %)

Eb: 41°/0,5 mm Hg
delta$_F$: - 63 ppm delta H: 6,7 ppm (1H); 7,03 ppm (1H) J: 8,5 Hz, 7,3 ppm (1H)

## 10

### Exemple 18

On procède comme à l'exemple 16 en remplaçant la méthyl-2 aniline par 10 g de méthoxy-3 aniline (0,08 mole).

Après 3 heures de réaction, on obtient:

- 0,8 de ditrifluorométhyl-2,6 méthoxy-3 aniline (rendement 4 %)
  delta$_F$: - 53,3 ppm; - 60,8 ppm delta$_H$: 7,53 ppm (d); 6,37 ppm (d) J = 8,5 Hz 3,87 ppm (3H, s)
- 3,3 g de méthoxy-3 trifluorométhyl-2 aniline (rendement 21 %)
  delta$_F$: - 53,2 ppm
- 3,7 de méthoxy-5 trifluorométhyl-2 aniline (rendement 24 %)
  delta$_F$: - 60,6 ppm
- 3,7 de méthoxy-3 trifluorométhyl-4 aniline (rendement 24 %)
  delta$_F$: - 60 ppm

### Exemple 19

On procède comme à l'exemple 16 en remplaçant la méthyl-2 aniline par 10 g de chloro-2 aniline (0,08 mole) et en thermostatant le flacon à 50°C.

Après 4 heures de réaction, on obtient:

- 1 g de chloro-2 ditrifluorométhylaniline (rendement 5 %)
- 3,2 de chloro-2 trifluorométhyl-6 aniline (rendement 21 %)
  delta$_F$: - 62 ppm
- 3,2 g de chloro-2 trifluorométhyl-4 aniline (rendement 21 %)
  delta$_F$: - 60 ppm

### Exemple 20

On procède comme à l'exemple 19 en remplaçant la chloro-2 aniline par 10 g de chloro-3 aniline (0,08 mole).

Après 3 heures de réaction, on obtient:

- 2,3 de chloro-5 trifluorométhyl-2 aniline (rendement 15 %)
  delta$_F$: - 62 ppm delta$_H$ = 6,73 ppm (2H, m); 7,27 ppm (1H, s)
- 1,8 de chloro-3 trifluorométhyl-2 aniline (rendement 12 %)
  delta$_F$: - 54 ppm
- 2,1 de chloro-3 trifluorométhyl-4 aniline (rendement 14 %)
  delta$_F$: - 60 ppm

### Exemple 21

On procède comme à l'exemple 14 en remplaçant l'aniline par 10 g de phényl-4 aniline (0,05 mole) et en remplaçant la méthyl-2 pyridine par 10 ml de diméthyl-2,6 pyridine.

Après 3 heures de réaction on obtient 1,55 g de phényl-4 trifluorométhyl-2 aniline (rendement 11 %) – delta$_F$: - 62 ppm

**Exemple 22**

On procède comme à l'exemple 21 en remplaçant la phényl-4 aniline par 10 g de phénoxy-4 aniline (0,053 mole).
Après 3 heures de réaction on obtient:

- 2,2 g de phénoxy-4 trifluorométhyl-2 aniline (rendement 16 %)
  delta$_F$: - 63 ppm

**Exemple 23**

On procède comme à l'exemple 21 en remplaçant la phényl-4 aniline par 10 g d'amino-1 naphtalène (0,07 mole).
Après 3 heures de réaction, on obtient un mélange de:

- amino-1 trifluorométhyl-2 naphtalène (rendement 30 %)
  delta$_F$: - 62 ppm
- amino-1 trifluorométhyl-4 naphtalène (rendement 20 %)
  delta$_F$: - 59 ppm

**Exemple 24**

On procède comme à l'exemple 16 en remplaçant la méthyl-2 aniline par 10 g de diamino-1,2 benzène.
Après 3 heures de réaction on obtient:

- 0,9 g de diamino-2,3 ditrifluorométhyl-1,4 benzène (rendement 4 %)
  delta$_F$: - 61,6 ppm (s); delta$_H$: 6,97 ppm (s)
- 0,7 g de diamino-1,2 ditrifluorométhyl-3,4 benzène (rendement 3 %)
  delta$_F$: - 55,2 ppm; delta$_H$: 7,15 ppm (1H, d); J$_{H-H}$ = 8,5 Hz
- 1,6 g de diamino-1,2 trifluorométhyl-3 benzène (rendement 10 %)
  delta$_F$: - 62 ppm
- 4,4 g de diamino-1,2 trifluorométhyl-4 benzène (rendement 27 %)
  delta$_F$: - 61 ppm

**Exemple 25**

On procède comme à l'exemple 24 en remplaçant le diamino-1,2 benzène par 10 g de diamino-1,3 benzène.
Après 3 heures de réaction on obtient:

- 1,6 g de diamino-1,3 ditrifluorométhyl-2,4 benzène (rendement 7 %)
- 1,4 g de diamino-1,5 ditrifluorométhyl-2,4 benzène (rendement 6 %)
- 2,6 g de diamino-1,3 trifluorométhyl-2 benzène (rendement 16 %)
- 1,1 g de diamino-1,3 trifluorométhyl-4 benzène (rendement 7 %)

**Exemple 26**

On procède comme à l'exemple 21 en remplaçant la phényl-4 aniline par 10 g de diamino-1,3 benzène (0,092 mole).
Après 2 heures de réaction on obtient:

- 3,8 g de diamino-1,3 ditrifluorométhyl-2,4 benzène (rendement 17 %)
  delta$_F$: - 54 ppm; - 60,5 ppm delta$_H$: 7,27 ppm; 5,98 ppm; J HH = 8,25 Hz
- 1,6 de diamino-1,5 ditrifluorométhyl-2,4 benzène (rendement 7 %)
  delta$_F$: - 60,7 ppm; delta$_H$: 7,5 ppm, 5,95 ppm
- 0,5 g de diamino-1,3 trifluorométhyl-2 benzène (rendement 3 %)
  delta$_F$: - 54 ppm
- 1,3 g de diamino-1,3 trifluorométhyl-4 benzène (rendement 8 %)
  delta$_F$: - 61,2 ppm

**Exemple 27**

On procède comme à l'exemple 16 en remplaçant la méthylaniline par 10 g de N,N diéthylaniline et la méthyl-2 pyridine par la pyridine.
Après 3 heures de réaction on obtient 5,3 g (rendement 36 %) d'un mélange équimoléculaire de:

- trifluorométhyl-2 N,N diéthylaniline
  delta$_F$ = - 58 ppm
- trifluorométhyl-4 N,N-diéthylaniline
  delta$_F$: - 60 ppm

**Exemple 28**

On procède comme à l'exemple 14 en remplaçant le bromotrifluorométhane par 30 g d'iodopentafluoroéthane. La pression descend en-dessous de la pression atmosphérique.
Après 3 heures de réaction on obtient 3,1 g d'un mélange équimoléculaire de:

- pentafluoroéthyl-2 aniline (rendement 14 %)
  delta$_F$: - 84 ppm (3F); - 113 ppm (2F)
- pentafluoroéthyl-4 aniline
  delta$_F$: - 85 ppm (3F); - 113,6 ppm (2F)

**Exemple 29**

Dans un erlenmeyer on introduit 10 g d'aniline, 25 ml de diméthylformamide, 1 g de zinc, 10 ml de diméthyl-2,6 pyridine et 6 g de soude en poudre. L'atmosphère dans l'erlenmeyer est constituée d'un mélange argon/dioxyde de soufre 50/50. On ajoute sous agitation 35 g d'iodure de perfluorobutyle en maintenant la température à 10°C. On ajoute ensuite 50 g de glace, 15 ml d'acide chlorhydrique concentré. Le mélange est extrait à l'éther, on obtient 20 g d'un mélange équimoléculaire (rendement 60 %) de:

— perfluorobutyl-2 aniline
  $\delta_F$: - 82 ppm (3F) - 111 ppm - 124 ppm - 127ppm
— perfluorobutyl-4 aniline
  $\delta_F$: - 82 ppm (3F) - 111 ppm - 124 ppm - 127 ppm

## Exemple 30

On procède comme à l'exemple 13 en remplaçant le zinc par 1 g de cadmium.

Après 3 heures de réaction et extraction à l'éther, on obtient:

1) 3,1 g de trifluorométhyl-2 aniline (rendement 18 %)
2) 1,4 g de trifluorométhyl-4 aniline (rendement 8 %)

## Exemple 31

On procède comme à l'exemple 13 en remplaçant le zinc par 1 g de fer.

Après 3 heures de réaction et extraction à l'éther, on obtient:

1) 1 g de trifluorométhyl-2 aniline (rendement 6 %)
2) 0,5 g de trifluorométhyl-4 aniline (rendement 3 %)

## Exemple 32

On procède comme à l'exemple 13 en remplaçant le zinc par 1 g de manganèse.

Après 3 heures de réaction et extraction à l'éther, on obtient:

1) 0,9 g de trifluorométhyl-2 aniline (rendement 5 %)
2) 0,4 g de trifluorométhyl-4 aniline (rendement 2 %)

## Exemple 33

Dans un appareil en verre épais, on introduit 40 g d'aniline, 100 ml de diméthylformamide, 40 ml de méthyl-2 pyridine et 4 g de zinc. L'appareil est thermostaté à 20°C. On y fait le vide puis on introduit 20 g de dioxyde de soufre. Le mélange est agité pendant deux heures sous une pression de bromotrifluorométhane qui est maintenue entre 7,5 et 6,5 atmosphères. La réaction est exothermique: la température atteint 70°C après 1/4 heure puis diminue peu à peu.

L'appareil est ensuite ouvert. Le mélange réactionnel est versé dans 200 g de glace et 60 ml d'acide chlorhydrique concentré. Le mélange est extrait à l'éther. On obtient par distillation:

1) 20 g de trifluorométhyl-2 aniline (rendement 29 %)
2) 10 g de trifluorométhyl-4 aniline (rendement 14 %)

## Exemple 34

On procède comme à l'exemple 13 en introduisant:

— 10 g (0,093 mole) de méthyl-3 aniline
— 25 ml de diméthylformamide
— 10 ml de méthyl-2 pyridine
— 6 g de métabisulfite de sodium
— 1 g de zinc

Après 3 heures de réaction et extraction à l'éther, on obtient 9,9 g d'un mélange qui est séparé par chromatographie en phase gazeuse. On obtient:

1) méthyl-3 ditrifluorométhylaniline (rendement 4 %)
2) méthyl-5 trifluorométhyl-2 aniline (rendement 12 %)
   $\delta_F$: - 61 ppm $\delta_H$: 7,3 ppm (1H, d, J: 8,5 Hz) 6,57 ppm (2H, d) 2,25 ppm (3H, s)
3) méthyl-3 trifluorométhyl-2 aniline (rendement 18 %)
   $\delta_F$: - 53,5 ppm (q) $\delta_H$: 7,07 ppm (1H, t, J: 8,5 Hz) 6,5 ppm (2H, m) 2,33 ppm (3H, q, $J_{HF}$: 3,3 Hz)
4) méthyl-3 trifluorométhyl-4 aniline (rendement 18 %)
   $\delta_F$: - 58 ppm (s large) $\delta_H$: 7,37 ppm (1H, d, J: 8,5 Hz) 6,43 ppm (2H, m) 2,33 ppm (3H, s large)

## Exemple 35

On procède comme à l'exemple 13 en introduisant:

— 10 g d'amino-3 pyridine
— 25 ml de diméthylformamide
— 6 g de métabisulfite de sodium
— 1 g de zinc

Après 3 heures de réaction on obtient un mélange de:

1) 1 % d'amino-3 trifluorométhyl-2 pyridine
   $\delta_F$: - 64,6 ppm; $\delta_H$: 7,04 ppm (1H, d, d, J: 8,5 Hz 1,5 Hz)
2) 3 % d'amino-5 trifluorométhyl-2 pyridine

deltaF: - 64,6 ppm; deltaH: 6,93 ppm (1H, d, d, J: 8,25 Hz 2,5 Hz) 7,39 ppm (1H, d, J: 8,25 Hz) 8,03 ppm (1H, d, J: 2,5 Hz)

## Exemple 36

On procède comme à l'exemple 4 en remplaçant l'oxyde de biphényle par 10 g de phénol.

Après 4 heures de réaction, extraction à l'éther on obtient:

1) 3,5 g de trifluorométhyl-2 phénol Eb 147 - 148°C (rendement 20 %)
   deltaF: - 61,6 ppm; deltaH: 7,48 ppm (2H, d) 7 ppm (2H, t)
   Le résidu est purifié par chromatographie en phase gazeuse, il contient, outre le phénol non réagi:
2) 1,7 g de trifluorométhyl-4 phénol (rendement 10 %)
   deltaF: - 60,3 ppm; deltaH: 7,53 ppm (2H, d) 6,93 ppm (2H, d, J: 8,5 Hz)

## Exemple 37

On procède comme à l'exemple 36 en remplaçant le phénol par par 10 g de chloro-3 phénol.

Après 4 heures de réaction et extraction à l'éther on obtient:

1) 2,6 g de chloro-3 trifluorométhyl-2 phénol (rendement 17 %)
   deltaF: - 55 ppm; deltaH: 7,23 ppm (1H, t, J: 8,5 Hz) 6,93 ppm (2H, m)
2) 2,9 de chloro-5 trifluorométhyl-2 phénol (rendement 19 %)
   deltaF - 60,3 ppm; deltaH: 7,4 ppm (1H, d, J: 8,5 Hz) 6,93 ppm (2H, m)
3) 1,8 g de chloro-3 trifluorométhyl-4 phénol (rendement 12 %)
   deltaF - 62 ppm; deltaH 7,53 ppm (1H, d, J: 8,5 Hz) 6,87 ppm (2H, m)

## Exemple 38

On procède comme à l'exemple 36 en remplaçant le phénol par par 10 g de chloro-2 phénol.

Après 4 heures de réaction et extraction à l'éther on obtient:

1) 1,4 g de chloro-6 trifluorométhyl-2 phénol (rendement 9 %)
   deltaH 7,37 ppm (2H, d) 6,63 ppm (1H, t, J: 8,5 Hz) deltaF - 62,6 ppm (s)
2) 0,9 de chloro-2 trifluorométhyl-4 phénol (rendement 6 %)
   deltaH 7,46 ppm; (1H, d, J: 1 Hz) 7,27 ppm (1H, dxd, J: 8,5 Hz; 1 Hz) 6,63 ppm (1H, d, J: 8,5 Hz);
   deltaF: - 61 ppm

## Exemple 39

On procède comme à l'exemple 36 en remplaçant le phénol par par 10 g de méthyl-3 phénol.

Après 4 heures de réaction et extraction à l'éther on obtient:

1) 4,2 g de méthyl-3 trifluorométhyl-2 phénol (rendement 26 %)
   Eb 23 mm Hg 77 - 79°C deltaF: - 53,3 ppm (q) deltaH: 7,27 ppm (1H, dxd, J: 8 Hz, J: 9 Hz); 6,8 ppm (2 H, d); 2,43 ppm (3H, q, JHF: 3 Hz)
2) 2,6 g de méthyl-5 trifluorométhyl-2 phénol (rendement 16 %)
   Eb 23 mm Hg 84 - 86°C deltaF: - 60 ppm deltaH: 7,4 ppm (1H, d, J: 8,5 Hz) 6,8 ppm (2H, d) 2,27 ppm (3H, s)
   On purifie par chromatographie en phase vapeur.
3) 3,9 g de méthyl-3 trifluorométhyl-4 phénol (rendement 24 %)
   deltaF: - 59 ppm (s large); deltaH: 7,43 ppm (1H, d, J: 8,5 Hz) 6,7 ppm (2H, m) 2,37 ppm (3H, s large)

## Exemple 40

On procède comme à l'exemple 36 en remplaçant le phénol par 10 g de méthoxy-3 phénol.

Après 3 heures de réaction et extraction à l'éther on obtient:

1) 3,9 de méthoxy-3 trifluorométhyl-2 phénol (rendement 25 %)
   deltaF: - 53,8 ppm; deltaH: 7,33 ppm (1H, t, J: 8,5 Hz) 6,56 ppm (2H, d) 3,87 ppm (3H, s)
2) 3,9 g de méthoxy-5 trifluorométhyl-2 phénol (rendement 25 %)
   deltaF: - 59 ppm; deltaH: 7,43 ppm (1H, d, J: 8,5 Hz) 6,53 ppm (2H, m) 3,77 ppm (3H, s)
3) 3,9 de méthoxy-3 trifluorométhyl-4 phénol (rendement 25 %)
   deltaF: - 61 ppm; deltaH: 7,43 ppm (1H, d, J: 8,5 Hz) 6,36 ppm (1H, m) 3,83 ppm (3H, s)

## Exemple 41

On procède comme à l'exemple 36 en remplaçant le phénol par 10 g de diméthoxy 1,3 benzène.

Après 4 heures de réaction et extraction à l'éther on obtient:

1) 3,3 g de diméthoxy-2,4 trifluorométhylbenzène (rendement 23 %)
   deltaH: 7,4 ppm (1H, d, H: 8,5 Hz) 6,8 ppm (1H, s large) 6,45 ppm (1H, m) 3,83 ppm (3H, s) 3,78 ppm (3H, s)
   deltaF: - 61,3 ppm (s)
2) 0,7 g de diméthoxy-2,6 trifluorométhylbenzène (rendement 4 %)
   deltaH: 7,45 ppm (1H, t) 6,63 ppm (2H, d, J: 8,5 Hz) 3,88 ppm (6H, s) deltaF: - 54 ppm (s)

## Exemple 42

Dans un flacon en verre épais, on introduit 10 g de dihydroxy-1,3 benzène, 25 ml de diméthylformamide, 1 g de zinc et 10 ml de méthyl-2 pyridine.

Le flacon est placé dans un appareil de Parr.

On fait le vide dans le flacon; on introduit 8 g de dioxyde de soufre. Le flacon est agité pendant 3 heures sous une pression de bromotrifluorométhane qui est maintenue entre 3,7 et 2,4 atmosphéres. La réaction est exothermique. Le flacon est ensuite ouvert, on ajoute 50 g de glace, 15 ml d'acide chlorhydrique concentré. Le mélange est extrait à l'éther. On obtient un mélange de:

1) 1 g de dihydroxy-1,3 trifluorométhyl-2 benzène (rendement 6 %)
   delta$_F$: - 53,3 ppm
2) 2,8 de dihydroxy-1,3 trifluorométhyl-4 benzène (rendement 17 %)
   delta$_F$: - 58,8 ppm

## Exemple 43

On procède comme à l'exemple 42 en remplaçant le dihydroxybenzène par 10 g (0,02 mole) d'amino-3 phénol.

Après 3 heures de réaction et extraction à l'éther, on obtient un mélange qui est purifié par chromatographie sur plaques de silice avec comme éluant un mélange benzène-acétate d'éthyle 90/10:

1) 0,5 g de ditrifluorométhyl amino-3 phénol (rendement 3 %)
2) 2,8 d'amino-3 trifluorométhyl-2 phénol (rendement 17 %)
   delta$_F$: - 54,5 ppm PF 121°C
3) 2,8 g d'amino-3 trifluorométhyl-4 phénol (rendement 17 %)
   delta$_F$: - 61 ppm PF 101°C
4) 2,8 d'amino-5 trifluorométhyl-2 phénol (rendement 17 %)
   delta$_F$: - 60,6 ppm PF 131°C

## Exemple 44

On procède comme à l'exemple 43 en remplaçant l'amino-3 phénol par 10 g d'amino-2 phénol.

Après 3 heures de réaction et extraction à l'éther, on obtient:

1) 2 g d'amino-2 ditrifluorométhylphénol (rendement 9 %)
2) 3,1 d'amino-2 trifluorométhyl-3 phénol (rendement 19 %)
   delta$_F$: - 62 ppm
3) 4,8 d'amino-2 trifluorométhyl-5 phénol (rendement 30 %)
   delta$_F$: - 59 ppm

## Exemple 45

On procède comme à l'exemple 36 en remplaçant le bromotrifluorométhane par 40 g d'iodo-pentafluoroéthane et en thermostatant le flacon à 20°C.

Après 3 heures de réaction et extraction à l'éther, on obtient:

1) 1,35 g de pentafluoroéthyl-2 phénol (rendement 6 %)
   delta$_F$: - 83,7 ppm (3F) - 111,7 ppm (2F)
2) 1,6 de pentafluoroéthyl-4 phénol (rendement 7 %)
   delta$_F$: - 84,3 ppm (3F) - 113,3 ppm (2F)

## Exemple 46

Dans un erlenmeyer, on introduit 2 g de phénol, 5 ml de diméthylformamide, 2 ml de méthyl-2 pyridine, 0,2 g de zinc, 1 g de métabisulfite de sodium.

L'atmosphère dans l'erlenmeyer est constituée par un mélange argon/dioxyde de soufre 50/50.

On ajoute sous agitation 9 g d'iodure de perfluorohexyle. On ajoute ensuite 10 g de glace, 5 ml d'acide chlorhydrique concentré. Le mélange est extrait à l'éther; on obtient après purification:

1) 1,2 de perfluorohexyl-2 phénol (rendement 14 %)
   delta$_F$: - 80 ppm (3F) - 107 ppm (2F) - 121 ppm (6F) - 125 ppm (2F)
   delta$_H$: 7,36 ppm (2H, d) 7 ppm (2H, t, J: 8,5 Hz)
2) 1,2 g de perfluorohexyl-4 phénol (rendement 14 %)
   delta$_F$: - 80 ppm (3F) - 108 ppm (2F) - 121 ppm (6F) - 125 ppm (2F)
   delta$_H$ 7,43 ppm (2H, d) 7,03 (2H, d) J: 8,5 Hz

## Exemple 47

On procède comme à l'exemple 4 en remplaçant l'oxyde de biphényle par 10 g de terbutyl-4 phénol.

Après 3 heures de réaction et extraction à l'éther on obtient:

— 3,6 g de terbutyl-4 trifluorométhyl-2 phénol F 49° (rendement 24 %)
  delta$_F$: - 60 ppm; delta$_H$: 7,55 ppm (1H, s, large) 7,33 ppm (1H, d) 6,8 ppm (1H, d, J: 8,5 Hz) 1,3 ppm 9 H (s)

## Exemple 48

On procède comme à l'exemple 4 en remplaçant l'oxyde de biphényle par 10 g de terbutyl-3 phénol.

Après 3 heures de réaction et extraction à l'éther on obtient:

1) 0,4 g de terbutyl-3 trifluorométhyl-2 phénol (rendement 3 %)
deltaF: - 50,2 ppm
2) 0,6 de terbutyl-3 trifluorométhyl-4 phénol (rendement 4 %)
deltaF: - 50,5 ppm
3) 2,8 de terbutyl-5 trifluorométhyl-2 phénol (rendement 19 %)
deltaF: - 61,3 ppm

**Exemple 49**

On procède comme à l'exemple 4 en remplaçant l'oxyde de biphényle par 10 g d'hydroxy-2 pyridine.

Après 3 heures de réaction et extraction à l'éther on obtient:

1) 3,4 g d'hydroxy-2 trifluorométhyl-6 pyridine F 52° (rendement 20 %)
deltaF: - 65,3 ppm; deltaH: 7,93 ppm (1H, d large J: 7 Hz) 7,63 ppm (1H, d large J: 7 Hz) 6,37 ppm (1H, t)

**Revendications**

1. Procédé de préparation de dérivés aromatiques perfluoralkylés caractérisé en ce que, dans une première étape, on met en présence un dérivé aromatique du dioxyde de soufre, un métal choisi parmi le zinc, l'aluminium, le manganèse et le cadmium dans un solvant aprotique polaire; dans une deuxième étape, on ajoute un bromure ou un iodure de perfluoroalkyle éventuellement en mélange avec du dioxyde de soufre et on maintient à une température comprise entre-20°C et 115°C.

2. Procédé selon la revendication 1, caractérisé en ce que dérivé aromatique répond à la formule générale (I):

$$Ar\text{--}(\text{--}R)n \tag{I}$$

dans laquelle:

— Ar représente un radical aromatique mono ou polycyclique, hétérocyclique,
— R représente au moins un substituant choisi parmi l'hydrogène, le chlore, le brome, les radicaux alkyles linéaires, ramifiés, cycliques saturés ou insaturés éventuellement substitués, éther, alcoxy éventuellement substitués, aryl éventuellement substitué, aryloxy, alkylthio, arylthio, amino, hydroxy, carboxylate, acyloxy, ester, amido, nitrile, acide,
— n est un nombre entier égal à 1, 2, 3.

3. Procédé selon la revendication 2, caractérisé en ce que, dans la formule générale (I)

— A représent un radical aromatique monocyclique,

— R représente un radical amino hydroxy.

4. Procédé selon la revendication 1, caractérisé en ce que le bromure ou l'iodure de perfluroalkyle répond à la formule générale (II)

$$C_nF_{2n+1}X \tag{II}$$

dans laquelle:

— n est un nombre entier compris entre 1 et 12,
— X représente Br ou I.

5. Procédé selon la revendication 4, caractérisé en ce que, dans la formule générale (II), lorsque n est égal à 1, X représente le brome.

6. Procédé selon la revendication 4, caractérisé en ce que dans la formule générale (II), lorsque n est supérieur à 1, X représente l'iode.

7. Procédé selon la revendication 1, caractérisé en ce que le métal choisi est le zinc.

8. Procédé selon la revendication 1, caractérisé en ce que le solvant aprotique polaire est choisi parmi le diméthylformamide, le diméthylsulfoxyde, l'acétonitrile, l'hexaméthylphosphoramide le diméthylacétamide, la N-météylpyrolidone.

9. Procédé selon la revendication 1, caractérisé en ce que, au cours de la première étape, on ajoute un base minérale et/ou une pyridine.

10. Procédé selon la revendication 9, caractérisé en ce que, au cours de la première étape, on ajoute un pyridine éventuellement substituée par au moins un groupe méthyle.

11. Procédé selon la revendication 9, caractérisé en ce que la base minérale est le métabisulfite de sodium.

12. Procédé selon la revendication 1, caractérisé en ce que le rapport molaire du métal au dérivé aromatique de formule (I) est compris entre 0,05 et 1 et de préférence entre 0,10 et 0,20.

13. Procédé selon la revendication 1, caractérisé en ce que le rapport molaire de l'halogénure de perfluoroalkyle au dérivé aromatique de formule (I) est supérieur ou égal à 1.

14. Procédé selon la revendication 1, caractérisé en ce que le rapport molaire du dioxyde soufre au dérivé aromatique de formule (I) est compris entre 0,05 et 1,5.

15. Procédé selon la revendication 9, caractérisé en ce que le rapport molaire de la base minérale au dérivé aromatique de formule (I) est compris entre 0,4 et 1.

16. Procédé selon la revendication 9, caractérisé en ce que le rapport molaire de la pyridine au dérivé aromatique de formule (I) est compris entre 0,5 et 1,5.

17. Procédé selon l'une quelconque des revendications précédentés, caractérisé en ce que la température de mise en oeuvre de la réaction est comprise entre 0 et 90°C.

**Patentansprüche**

1. Verfahren zur Herstellung aromatischer Perfluoralkylverbindungen, dadurch gekennzeichnet, daß man in einem ersten Schritt eine aromatische Verbindung, Schwefeldioxid und ein Metall ausgewählt aus Zink, Aluminium, Mangan und Cadmium in einem aprotischen polaren Lösungsmittel umsetzt und in einem zweiten Schritt ein Perfluoralkylbromid oder -jodid gegebenenfalls im Gemisch mit Schwefeldioxid zugibt und auf einer Temperatur zwischen -20°C und 115°C hält.

2. Verfahren nach Anspruch 1, dadurch gekennzeichnet, daß die aromatische Verbindung der allgemeinen Formel (I) entspricht

$$Ar-(-R)_n \qquad (I)$$

in der:

- Ar einen aromatischen mono- oder polycyclischen oder heterocyclischen Rest darstellt,
- R wenigstens einen Substituenten ausgewählt aus Wasserstoff, Chlor, Brom, den linearen, verzweigten, cyclischen gesättigten oder ungesättigten Alkylresten, die gegebenenfalls substituiert sind, Ethern, Alkoxyresten, gegebenenfalls substituiert, Arylresten, gegebenenfalls substituiert, Aryloxy-, Alkylthio-, Arylthio-, Amino-, Hydroxy-, Carboxylat-, Acyloxy-, Ester-, Amido-, Nitril-, Säureresten darstellt,
- n eine ganze Zahl und gleich 1, 2 oder 3 ist.

3. Verfahren nach Anspruch 2, dadurch gekennzeichnet, daß in der allgemeinen Formel (I)

- Ar einen aromatischen monocyclischen Rest darstellt,
- R einen Amino- oder Hydroxyrest darstellt.

4. Verfahren nach Anspruch 1 dadurch gekennzeichnet, daß das Perfluoralkylbromid oder -jodid der allgemeinen Formel (II) entspricht

$$C_nF_{2n+1}X \qquad (II)$$

in der:

- n eine ganze Zahl zwischen 1 und 12 ist,
- X Br oder J darstellt.

5. Verfahren nach Anspruch 4, dadurch gekennzeichnet, daß in der allgemeinen Formel (II) X Brom darstellt, wenn n gleich 1 ist.

6. Verfahren nach Anspruch 4, dadurch gekennzeichnet, daß in der allgemeinen Formel (II) X Jod darstellt, wenn n größer ist als 1.

7. Verfahren nach Anspruch 1, dadurch gekennzeichnet, daß das gewählte Metall Zink ist.

8. Verfahren nach Anspruch 1, dadurch gekennzeichnet, daß das aprotische polare Lösungsmittel ausgewählt wird aus Dimethylformamid, Dimethylsulfoxid, Acetonitril, Hexamethylphosphoramid, Dimethylacetamid, N-Methylpyrrolidon.

9. Verfahren nach Anspruch 1, dadurch gekennzeichnet, daß im Verlauf des ersten Schrittes eine Mineralbase und/oder ein Pyridin zugegeben wird.

10. Verfahren nach Anspruch 9, dadurch gekennzeichnet, daß im Verlauf des ersten Schrittes ein Pyridin zugegeben wird, das gegebenenfalls mit wenigstens einer Methylgruppe substituiert ist.

11. Verfahren nach Anspruch 9, dadurch gekennzeichnet, daß die Mineralbase Natriummetabisulfit ist.

12. Verfahren nach Anspruch 1, dadurch gekennzeichnet, daß das molare Verhältnis des Metalls zur aromatischen Verbindung der Formel (I) zwischen 0,05 und 1 und vorzugsweise zwischen 0,10 und 0,20 liegt.

13. Verfahren nach Anspruch 1, dadurch gekennzeichnet, daß das molare Verhältnis des Perfluoralkylhalogenids zur aromatischen Verbindung der Formel (I) größer oder gleich 1 ist.

14. Verfahren nach Anspruch 1, dadurch gekennzeichnet, daß das molare Verhältnis des Schwefeldioxids zur aromatischen Verbindung der Formel (I) zwischen 0,05 und 1,5 liegt.

15. Verfahren nach Anspruch 9, dadurch gekennzeichnet, daß das molare Verhältnis der Mineralbase zur aromatischen Verbindung der Formel (I) zwischen 0,4 und 1 liegt.

16. Verfahren nach Anspruch 9, dadurch gekennzeichnet, daß das molare Verhältnis von Pyridin zur aromatischen Verbindung der Formel (I) zwischen 0,5 und 1,5 liegt.

17. Verfahren nach einem der vorhergehenden Ansprüche, dadurch gekennzeichnet, daß die Umsetzungstemperatur der Reaktion zwischen 0 und 90°C liegt.

## Claims

1. Process for the preparation of perfluoroalkylated aromatic derivatives, characterized in that, in a first stage, an aromatic derivative, sulphur dioxide, a metal chosen from zinc, aluminium, manganese and cadmium are brought into contact in a polar aprotic solvent; in a second stage, a perfluoroalkyl bromide or iodide, optionally mixed with sulphur dioxide, is added and a temperature of between -20°C and 115°C is maintained.

2. Process according to Claim 1, characterized in that the aromatic derivative corresponds to the general formula (I):

$$Ar-(-R)_n \qquad (I)$$

in which:

Ar denotes a mono- or polycyclic or heterocyclic aromatic radical,
R denotes at least one substituent chosen from hydrogen, chlorine, bromine, saturated or unsaturated, optionally substituted, linear, branched or cyclic alkyl radicals, ether, optionally substituted alkoxy, optionally substituted aryl, aryloxy, alkylthio, arylthio, amino, hydroxy, carboxylate, acyloxy, ester, amido, nitrile and acid radicals, and
n is an integer equal to 1, 2 or 3.

3. Process according to Claim 2, characterized in that, in the general formula (I)

Ar denotes a monocyclic aromatic radical, and
R denotes an amino or hydroxy radical.

4. Process according to Claim 1, characterized in that the perfluoroalkyl bromide or iodide corresponds to the general formula (II)

$$C_nF_{2n+1}X \qquad (II)$$

in which:

n is an integer from 1 to 12, and
X denotes Br or I.

5. Process according to Claim 4, characterized in that, in the general formula (II), when n is equal to 1, X denotes bromine.

6. Process according to Claim 4, characterized in that, in the general formula (II), when n is greater than 1, X denotes iodine.

7. Process according to Claim 1, characterized in that the metal chosen is zinc.

8. Process according to Claim 1, characterized in that the polar aprotic solvent is chosen from dimethylformamide, dimethyl sulphoxide, acetonitrile, hexamethylphosphoramide, dimethylacetamide and N-methylpyrrolidone.

9. Process according to Claim 1, characterized in that an inorganic base and/or a pyridine is, or are, added during the first stage.

10. Process according to Claim 9, characterized in that a pyridine, optionally substituted by at least one methyl group, is added during the first stage.

11. Process according to Claim 9, characterized in that the inorganic base is sodium metabisulphite.

12. Process according to Claim 1, characterized in that the molar ratio of the metal to the aromatic derivative of formula (I) is between 0.05 and 1 and, preferably, between 0.10 and 0.20.

13. Process according to Claim 1, characterized in that the molar ratio of the perfluoroalkyl halide to the aromatic derivative of formula (I) is higher than or equal to 1.

14. Process according to Claim 1, characterized in that the molar ratio of sulphur dioxide to the aromatic derivative of formula (I) is between 0.05 and 1.5.

15. Process according to Claim 9, characterized in that the molar ratio of the inorganic base to the aromatic derivative of formula (I) is between 0.4 and 1.

16. Process according to Claim 9, characterized in that the molar ratio of the pyridine to the aromatic derivative of formula (I) is between 0.5 and 1.5.

17. Process according to any one of the preceding claims, characterized in that the temperature at which the reaction is carried out is between 0 and 90°C.